# FASCICULE DE BREVET EUROPEEN

(11) **EP 1 036 554 B1**
(45) Date de publication et mention de la délivrance du brevet: **24.07.2002**
(21) Numéro de dépôt: 00400425.5
(22) Date de dépôt: 15.02.2000
(51) Int. Cl.: A61K 7/02, A61K 7/00

(54) **Composition et procédé de maquillage en relief des matières kératiniques**
Zusammensetzung und Verfahren zur erzeugung eines Relief Make-up auf keratinischen Materialien
Composition and process for forming a relief make-up on keratinic materials

(30) Priorité: 11.03.1999 FR 9903006
(43) Date de publication de la demande: 20.09.2000
(73) Titulaire: L'OREAL, 75008 Paris (FR)
(72) Inventeur: Ramin, Roland, 75014 Paris (FR)
(74) Mandataire: Kromer, Christophe

(56) Documents cités:
- FR-A- 2 757 049
- FR-A- 2 757 050
- FR-A- 2 757 052

## Description

La présente invention se rapporte à une composition cosmétique de maquillage granité des matières kératiniques comprenant un polymère filmogène et des particules, ainsi qu'un kit de maquillage comprenant ladite composition. L'invention a aussi pour objet un procédé cosmétique de maquillage des matières kératiniques. La composition selon l'invention peut être appliquée sur les ongles, la peau aussi bien du visage que du corps, y compris les lèvres, et sur les cheveux, notamment d'êtres humains. Plus spécialement, l'invention porte sur un vernis à ongles.

La composition de maquillage peut être un vernis à ongles, un fard à joue ou à paupières, un fond de teint, un produit de maquillage pour les lèvres ou un produit de maquillage du corps. La composition peut également être appliquée sur les accessoires de maquillage comme les faux ongles, faux cils, perruques ou encore sur des pastilles ou des patchs adhérents sur la peau ou les lèvres (du type mouches).

Les compositions de vernis à ongles comprennent généralement un polymère filmogène soit solubilisé dans un solvant organique, soit dispersé sous forme de particules dans un milieu aqueux, et une matière colorante, notamment un pigment. De tels vernis sont par exemple décrits dans les documents US-A-4158053 et FR-A-2578741.

Ces vernis à ongles, après l'application sur l'ongle d'une ou de plusieurs couches de la composition et après séchage, conduisent généralement à la formation d'un film lisse, continu et homogène, brillant ou mat. Certains films présentent aussi de bonnes propriétés cosmétiques telles qu'une bonne tenue et en particulier une bonne adhérence sur l'ongle, une bonne résistance à l'eau, aux frottements et aux chocs.

Avec l'évolution de la mode, les consommateurs, de plus en plus exigeants, recherchent de nouveaux produits de maquillage conférant des effets de maquillage originaux ou particuliers. Un besoin subsiste donc de disposer de produit de maquillage dont l'application sur un support comme les ongles, la peau ou les cheveux d'êtres humains conduit à un effet de maquillage différent de ceux des films lisses, continus et homogènes actuellement obtenus avec les produits disponibles sur le marché.

La présente invention a donc pour but de proposer une composition de maquillage permettant d'obtenir un maquillage en relief sur les matières kératiniques, tout en présentant une bonne tenue dans le temps. Le maquillage confère un aspect granité ou de "crépi" sur le support où il est appliqué.

Le demandeur a constaté qu'un nouveau type de maquillage des matières kératiniques pouvait être obtenu en utilisant une composition filmogène comprenant des fragments de film coloré particuliers.

De façon plus précise, l'invention a pour objet une composition cosmétique de maquillage comprenant dans un milieu cosmétiquement acceptable un polymère filmogène et une matière colorante, caractérisée par le fait que la matière colorante comprend un fragment de film de polymère coloré présentant deux faces sensiblement planes, ayant une épaisseur allant de 40 à 200 µm et dont la plus grande dimension va de 0,1 à 4 mm, le dit fragment étant insoluble dans le milieu de la composition.

Lorsque la composition est appliquée sur les matières kératiniques tels que les ongles, les fragments de films se répartissent facilement dans la couche déposée et se disposent bien dans l'épaisseur de la couche. Cette composition procure un film de maquillage présentant des parties plus épaisses que l'épaisseur moyenne du film, réparties de façon aléatoire à la surface du film. La surface du film de maquillage présente un relief discontinu, résistant aux frottements. On obtient ainsi un maquillage en relief original, aussi bien au toucher qu'à la vue, présentant une bonne adhérence sur le support maquillé et une bonne tenue.

Avantageusement cette composition peut être appliquée comme produit de surface, communément appelée "top coat" en terminologie anglosaxonne.

Un autre objet de l'invention est un procédé cosmétique de maquillage des matières kératiniques consistant à appliquer sur les matières kératiniques une composition telle que définie précédemment.

L'invention a aussi pour objet un procédé cosmétique de maquillage des matières kératiniques consistant à appliquer sur les matières kératiniques une première couche, appelée aussi couche de base, d'une première composition cosmétique comprenant dans un milieu cosmétiquement acceptable un premier polymère filmogène, puis à appliquer sur au moins une partie de la première couche une deuxième couche, appelée aussi couche de surface, d'une deuxième composition cosmétique comprenant, dans un milieu cosmétiquement acceptable, au moins un deuxième polymère filmogène et au moins un fragment de film de polymère coloré présentant deux faces sensiblement planes, ayant une épaisseur allant de 40 à 200 µm et dont la plus grande dimension va de 0,1 à 4 mm, le dit fragment étant insoluble dans le milieu de la composition, la première composition ne contenant pas de fragment de film de polymère comme présent dans la deuxième composition.

L'invention a également pour objet un kit de maquillage comprenant :
- une première composition cosmétique (de couche de base) comprenant dans un milieu cosmétiquement acceptable un premier polymère filmogène ;
- une deuxième composition cosmétique (de couche de surface) comprenant, dans un milieu cosmétiquement acceptable, au moins un deuxième polymère filmogène et au moins un fragment de film de polymère coloré présentant deux faces sensiblement planes, ayant une épaisseur allant de 40 à 200 µm et dont la plus grande dimension va de 0,1 à 4 mm, le dit fragment étant insoluble dans le milieu de la composition,
la première composition ne contenant pas de fragment de film de polymère comme présent dans la deuxième composition.

L'invention a également pour objet un support maquillé comprenant un maquillage susceptible d'être obtenu selon le procédé tel que défini précédemment et appliqué sur ledit support.

Le fragment de film de polymère coloré peut être obtenu à partir de polymères radicalaires, et notamment de polymères vinyliques tels que les polymères acryliques ou les polymères à base d'acétate de polyvinyle, les copolymères styrène-acrylique, vinyle/versatate, vinyle/éthylène, les terpolymères vinyle/versatate/acrylate ou vinyle/éthylène/chlorure. On utilise de préférence les copolymères styrène/acrylique.

La couleur du fragment de film n'est pas critique et peut être choisie parmi toutes les teintes possibles. On peut notamment utiliser un mélange de fragments de film de teintes différentes. La fragment de film peut également comporter une couleur différente sur chaque face, comme décrit dans le document EP-A-679698.

Avantageusement, l'épaisseur du fragment de film peut aller d'environ 70 µm à environ 130 µm, et mieux d'environ 90 µm à environ 110 µm.

La plus grande dimension du fragment de film, mesurée dans le plan passant par l'une des deux faces planes du fragment de film, peut aller de préférence de 0,1 mm à 2 mm, et mieux de 0,1 mm à 1 mm.

De préférence, le film fragmenté est mat et ne présente donc pas d'effet scintillant ou brillant. Avantageusement, le film fragmenté peut avoir une brillance, mesurée à l'aide d'un brillancemètre BYK-GARDNER à un angle de faisceau lumineux de 60°, inférieure ou égale à 15 (notamment de 3 à 15), et en particulier allant de 3 à 10.

Le fragment de film peut être coloré à l'aide de pigments ou de colorants. Comme pigment, on peut citer les pigments minéraux, les pigments organiques, les nacres, les poudres de métaux.

Le fragment de film peut en outre comprendre au moins une charge, ce qui permet notamment d'obtenir un film ayant les propriétés physico-chimiques souhaitées. Comme charge, on peut citer le carbonate de calcium, le carbonate de magnésium, le carbonate de chaux, le sulfate de barium, le talc, le kaolin.

Selon l'invention, le fragment de film coloré peut être présent, notamment dans la composition de surface, en une teneur allant de 0,5 % à 30 % en poids, par rapport au poids total de cette composition de surface, de préférence de 3 % à 20 % en poids, et mieux de 5 % à 15 % en poids.

Des fragments de film coloré utilisés selon l'invention sont notamment commercialisés sous la dénomination MONOCOLOR par la société QUADRA INDUSTRIES.

Selon l'invention, le milieu cosmétiquement acceptable des compositions selon l'invention peut comprendre un milieu aqueux ou un milieu solvant organique. En particulier les première et deuxième compositions peuvent comprendre, indépendamment l'une de l'autre, un milieu aqueux ou un milieu solvant organique.

Ainsi, selon une première variante de réalisation de l'invention, les première et deuxième compositions peuvent comprendre un milieu aqueux, identique ou différent.

Selon une deuxième variante de réalisation de l'invention, la première composition peut comprendre un milieu aqueux et la deuxième composition peut comprendre un milieu solvant organique.

Selon une troisième variante de réalisation de l'invention, la première composition peut comprendre un milieu solvant organique et la deuxième composition peut comprendre un milieu aqueux.

Selon une quatrième variante de réalisation de l'invention, les première et deuxième compositions peuvent comprendre un milieu solvant organique, identique ou différent.

Dans la présente demande, on entend par "polymère filmogène", un polymère apte à former à lui seul ou en présence d'un agent auxiliaire de filmification, un film isolable. Le polymère filmogène des compositions de base et de surface peut être solubilisé ou dispersé sous forme de particules dans le milieu cosmétiquement acceptable correspondant de chaque composition selon l'invention. Le polymère filmogène n'est donc pas présent sous forme d'un film déjà formé mais bien au contraire, sous une forme telle qu'il formera un film après l'application de la composition sur les matières kératiniques.

Parmi les polymères filmogènes utilisables dans les compositions de la présente invention, on peut citer les polymères synthétiques, de type radicalaire ou de type polycondensat, les polymères d'origine naturelle, et leurs mélanges. Les premier et deuxième polymères filmogènes des première et deuxième compositions peuvent être identiques ou différents.

Par polymère filmogène radicalaire, on entend un polymère obtenu par polymérisation de monomères à insaturation notamment éthylénique, chaque monomère étant susceptible de s'homopolymériser (à l'inverse des polycondensats). Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

On utilise de préférence des polymères filmogènes radicalaires anioniques, c'est-à-dire des polymères ayant au moins un monomère à groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique en dispersion aqueuse utilisable selon l'invention, on peut citer ceux vendus sous les dénominations NEOCRYL XK-90®, NEOCRYL A-1070®, NEOCRYL A-1090®, NEOCRYL BT-62®, NEOCRYL A-1079®, NEOCRYL A-523® par la société ZENECA, DOW LATEX 432® par la société DOW CHEMICAL.

On peut ainsi citer, parmi les polycondensats utilisables comme polymère filmogène, les polyuréthannes anioniques, cationiques, non-ioniques ou amphotères, les polyuréthanne-acryliques, les poly-uréthanne-polyvinylpyrrolidones, les polyester-polyuréthannes, les polyéther-polyuréthannes, les polyurées, les polyurée/polyuréthannes, et leurs mélanges.
Le polyuréthanne filmogène peut être, par exemple, un copolymère polyuréthanne, polyurée-uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Comme polymère filmogène polyuréthane en dispersion aqueuse utilisable selon l'invention, on peut notamment citer les polyester-polyuréthanes vendus sous les dénominations "AVALURE UR-405®", "AVALURE UR-410®", "AVALURE UR-425®", "SANCURE 2060®" par la société GOODRICH et les polyéther-polyuréthanes vendus sous les dénominations "SANCURE 878®" par la société GOODRICH, "NEOREZ R 970®" par la société ICI.

Parmi les polycondensats filmogènes, on peut également citer les polyesters, les polyesters amides, les polyesters à chaîne grasse, les polyamides, et les résines époxyesters, les résines résultant de la condensation de formaldéhyde avec une arylsulfonamide, les résines aryl-sulfonamide époxy.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine, Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques tels que la nitrocellulose, l'acétate de cellulose, l'acétobutyrate de cellulose, l'acétopropionate de cellulose, l'éthyl cellulose, et leurs mélanges.

Le polymère filmogène des compositions de base et de surface peut être généralement présent respectivement en une teneur allant de 1 % à 70 % en poids, par rapport au poids total de la composition, respectivement de base et de surface, et mieux allant de 10 % à 40 % en poids.

Comme solvant organique utilisable dans l'invention, on peut citer :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane,
- les composés cycliques aromatiques liquides à température ambiante tels que le toluène et le xylène ;
- les aldéhydes liquides à température ambiante tels que le benzaldéhyde, l'acétaldéhyde
- leurs mélanges.

Ces solvants conviennent plus particulièrement pour le maquillage des ongles : la composition constitue alors un vernis à ongles.

Dans chaque composition à milieu solvant organique, le solvant organique peut être présent en une teneur allant de 30 à 99 % en poids, par rapport au poids total de chaque composition, et de préférence de 60 % à 90 % en poids.

Lorsque les compositions pour la mise en oeuvre du procédé selon l'invention contiennent un milieu aqueux, ce dernier peut être constitué essentiellement d'eau ou bien encore d'un mélange hydroalcoolique et en particulier contenant des monoalcools inférieurs en C₁-C₅. La teneur en eau dans chaque composition à milieu aqueux peut aller de 30 à 99 % en poids, par rapport au poids total de chaque composition, et de préférence de 60 % à 90 % en poids.

Pour améliorer les propriétés filmogènes de la composition, notamment de la composition de base et/ou de surface selon l'invention, un agent auxiliaire de filmification peut être prévu.

Un tel agent auxiliaire de filmification peut être choisi parmi tous les composés connus de l'homme du métier comme étant susceptibles de remplir la fonction recherchée, et être notamment choisi parmi les agents plastifiants.

En outre, lorsque la composition, ou l'une des compositions de base et/ou de surface selon l'invention, comprend un polymère filmogène sous forme de particules dispersées dans le milieu correspondant de la composition, l'agent auxiliaire de filmification peut aussi être choisi parmi les agents de coalescence.

La composition de base et/ou de surface selon l'invention peuvent en outre comprendre des matières colorantes additionnelles, différentes du fragment de film décrit précédemment. La matière colorante peut notamment être choisie parmi les colorants (hydrosolubles ou liposolubles) et les matières colorantes pulvérulentes tels que les pigments, les nacres, les paillettes bien connues de l'homme du métier. En particulier, les matières colorantes pulvérulentes peuvent être choisies parmi celles ayant une taille de particule moyenne inférieure ou égale à 70 µm (notamment allant de 1 µm à 70 µm), et de préférence inférieure ou égale à 50 µm (notamment allant de 1 µm à 50 µm). Les matières colorantes additionnelles peuvent être présentes, dans chaque composition, en une teneur allant de 0,01 % à 15 % en poids, par rapport au poids de chaque composition. Ces matières colorantes peuvent permettre en particulier d'obtenir un film de maquillage lisse, contrairement au film de maquillage comprenant le fragment de film coloré défini précédemment.

Selon un mode préféré de réalisation du kit et du procédé de maquillage selon l'invention, la composition de base peut comprendre une matière colorante consistant en un colorant hydrosoluble ou liposoluble et/ou les matières colorantes pulvérulentes ayant une taille moyenne de particule inférieure ou égale à 70 µm, et mieux inférieure ou égale à 50 µm. En particulier, la composition de base peut être colorée et être apte à former un premier film coloré et la composition de surface peut comprendre un milieu sensiblement translucide de telle sorte que ledit milieu ne masque pas la couleur du fragment de film de polymère coloré. L'application de la composition de base sur les matières kératiniques puis de la composition de surface conduit alors à un maquillage faisant apparaître de manière distincte les fragments de film coloré, répartis de façon aléatoire sur la couche colorée obtenue avec la composition de base. On observe alors un contraste entre les couleurs des fragments de film et la couleur de la couche de base. Avantageusement, la composition de base a une couleur différente de celles des fragments de film coloré et éventuellement de celle du milieu de la composition de surface.

Les compositions de base et de surface selon l'invention peuvent en outre comprendre tout additif connu de l'homme du métier comme étant susceptible d'être incorporé dans une telle composition, tels que les agents épaississants, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition de base et celle de surface selon l'invention peuvent être préparées par l'homme du métier sur la base de ses connaissances générales et selon l'état de la technique.

Avantageusement, la composition de base et celle de surface sont conditionnées dans des compartiments ou récipients distincts, accompagnés de moyens d'application appropriés, identiques ou différents, tels que des pinceaux, des brosses, des plumes, des éponges.

La composition comprenant le fragment de film de polymère coloré peut être appliquée soit à l'une des extrémités de la couche de base, soit au milieu, ou encore de façon discontinue notamment sous forme de motifs géométriques, symétriques ou dissymétriques (par exemple sous forme de points, carrés, ronds, étoiles), répartis de façon aléatoire ou ordonnée, à contours précis ou flous.

L'invention est illustrée plus en détail dans les exemples suivants.

### Example 1 :

On a préparé un vernis à ongles ayant la composition suivante :
- Nitrocellulose 10 g
- Plastifiants et résine 15 g
- Silice pyrogénée (Aérosil® 200 de DEGUSSA) 1,5 g
- Particules blanches en forme de film fragmenté (MONOCOLOR WE® 150/000 de QUADRA) 10 g
- Acétate d'éthyle, acétate de butyle qsp 100 g

La composition comprend un milieu transparent dans lequel sont dispersées les particules de film fragmenté de couleur blanche.

Après application de la composition sur les ongles, on a obtenu un film de maquillage en relief présentant une répartition discontinue de particules blanches. Le maquillage a un aspect granité.

### Exemple 2 :

On a préparé deux compositions A et B de vernis à ongles suivantes :

### Composition de base A :

- Nitrocellulose 19 g
- N-éthyl o,p-toluènesulfonamide 6 g
- Acétyl citrate de tri-butyle 6 g
- Pigments noirs 1 g
- Hectorite 1,2 g
- Alcool isopropylique 8 g
- Acétate d'éthyle, acétate de butyle qsp 100 g

### Composition de surface B :

- Nitrocellulose 10 g
- Plastifiants et résine 15 g
- Silice pyrogénée (Aérosil® 200 de DEGUSSA) 1,5 g
- Particules blanches en forme de film fragmenté (MONOCOLOR WE® 150/000 de QUADRA) 3 g
- Particules roses en forme de film fragmenté (MONOCOLOR PEK® 426/000 de QUADRA) 7 g
- Acétate d'éthyle, acétate de butyle qsp 100 g

On a appliqué sur l'ongle une couche de base de la composition A puis après séchage une couche de surface de la composition B. On a obtenu un maquillage en relief de bonne tenue présentant un dépôt discontinu de particules blanches et roses sur un fond noir. Le maquillage obtenu a l'aspect d'un crépi.

## Revendications

1. Composition cosmétique de maquillage comprenant dans un milieu cosmétiquement acceptable un polymère filmogène et une matière colorante, **caractérisée par le fait que** la matière colorante comprend au moins un fragment de film de polymère coloré présentant deux faces sensiblement planes, ayant une épaisseur allant de 40 à 200 µm et dont la plus grande dimension va de 0,1 à 4 mm, le dit fragment étant insoluble dans le milieu de la composition.

2. Composition selon la revendication 1, **caractérisée par le fait que** le fragment de film a une épaisseur allant de 70 µm à 130 µm, et mieux de 90 µm à 110 µm.

3. Composition selon la revendication 1 ou 2, **caractérisée par le fait que** le fragment de film a une brillance, mesurée à l'aide d'un brillancemètre BYK-GARDNER à un angle de faisceau lumineux de 60°, inférieure ou égale à 15, et de préférence allant de 3 à 10.

4. Composition selon l'une quelconque des revendications précédentes, **caractérisée par le fait que** la plus grande dimension du fragment de film coloré va de 0,1 mm à 2 mm, et mieux de 0,1 mm à 1 mm.

5. Composition selon l'une quelconque des revendications précédentes, **caractérisée par le fait que** le fragment de film coloré est issu d'un film de polymère choisi dans le groupe formé par les polymères acryliques, les polymères d'acétate de polyvinyle, les copolymères styrène-acrylique, vinyle/versatate, vinyle/éthylène, les terpolymères vinyle/versatate/acrylate ou vinyle/éthylène/chlorure.

6. Composition selon l'une quelconque des revendications précédentes, **caractérisée par le fait que** le fragment de film est présent en une teneur allant de 0,5 % à 30 % en poids, par rapport au poids total de la composition, et de préférence de 3 % à 20 % en poids.

7. Composition selon l'une quelconque des revendications précédentes, **caractérisée par le fait qu'**elle comprend un milieu solvant organique ou un milieu aqueux.

8. Composition selon l'une quelconque des revendications précédentes, **caractérisée par le fait que** le polymère filmogène est choisi dans le groupe formé par les polymères radicalaires, les polycondensats et les polymères d'origine naturelle.

9. Composition selon l'une quelconque des revendications précédentes, **caractérisée par le fait que** le polymère filmogène est choisi dans le groupe formé par les polymères vinyliques, les polyuréthanes, les polyesters, les polymères cellulosiques.

10. Composition selon l'une quelconque des revendications précédentes, **caractérisée par le fait que** la teneur en polymère filmogène va de 1 % à 70 % en poids, par rapport au poids total de la composition, et de préférence de 10 % à 40 % en poids.

11. Composition selon l'une quelconque des revendications précédentes, **caractérisée par le fait qu'**elle comprend en outre une matière colorante additionnelle différente de celle du fragment de film coloré.

12. Composition selon la revendication 11, **caractérisée par le fait que** la matière colorante additionnelle est choisie parmi les colorants et les matières colorantes pulvérulentes ayant une taille de particules inférieure ou égale à 70 µm.

13. Composition selon l'une quelconque des revendications précédentes, **caractérisée par le fait qu'**elle comprend en outre au moins un additif choisi dans le groupe formé par les agents auxiliaires de filmification, les agents épaississants, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

14. Composition selon l'une quelconque des revendications précédentes, **caractérisée par le fait qu'**elle se présente sous la forme de vernis à ongles, de fard à joue ou à paupières, de fond de teint ou de produit de maquillage du corps.

15. Procédé cosmétique de maquillage des matières kératiniques consistant à appliquer sur les matières kératiniques au moins une couche d'une composition selon l'une quelconque des revendications précédentes.

16. Procédé cosmétique de maquillage des matières kératiniques, **caractérisé par le fait que** l'on applique sur les matières kératiniques :
- une première couche d'une première composition comprenant dans un milieu cosmétiquement acceptable un premier polymère filmogène,
- puis à appliquer sur au moins une partie de ladite première couche, une deuxième couche d'une deuxième composition comprenant au moins un deuxième polymère filmogène dans un milieu cosmétiquement acceptable, et au moins une matière colorante comprenant au moins un fragment de film de polymère coloré présentant deux faces sensiblement planes, ayant une épaisseur allant de 40 à 200 µm et dont la plus grande dimension va de 0,1 à 4 mm, le dit fragment étant insoluble dans le milieu de la composition,
la première composition ne comprenant pas de fragment de polymère comme présent dans la deuxième composition.

17. Procédé selon la revendication 16, **caractérisé par le fait que** le fragment de film a une épaisseur allant de 70 µm à 130 µm, et mieux de 90 µm à 110 µm.

18. Procédé selon la revendication 16 ou 17, **caractérisée par le fait que** le fragment de film a une brillance, mesurée à l'aide d'un brillancemètre BYK-GARDNER à un angle de faisceau lumineux de 60°, inférieure ou égale à 15, et de préférence allant de 3 à 10.

19. Procédé selon l'une quelconque des revendications 16 à 18, **caractérisée par le fait que** la plus grande dimension du fragment de film coloré va de 0,1 mm à 2 mm, et mieux de 0,1 mm à 1 mm.

20. Procédé selon l'une quelconque des revendications 16 à 19, **caractérisée par le fait que** le fragment de film coloré est issu d'un film de polymère choisi dans le groupe formé par les polymères acryliques, les polymères d'acétate de polyvinyle, les copolymères styrène-acrylique, vinyle/versatate, vinyle/éthylène, les terpolymères vinyle/versatate/acrylate ou vinyle/éthylène/chlorure.

21. Procédé selon l'une quelconque des revendications 16 à 20, **caractérisé par le fait que** le fragment de film est présent en une teneur allant de 0,5 % à 30 % en poids, par rapport au poids total de la deuxième composition, et de préférence de 3 % à 20 % en poids.

22. Procédé selon l'une quelconque des revendications 16 à 21, **caractérisé par le fait que** la première et/ou la deuxième composition comprennent un milieu solvant organique ou un milieu aqueux.

23. Procédé selon l'une quelconque des revendications 16 à 22, **caractérisé par le fait que** les premier et deuxième polymères filmogènes sont choisis, indifféremment l'un de l'autre, dans le groupe formé par les polymères radicalaires, les polycondensats et les polymères d'origine naturelle.

24. Procédé selon l'une quelconque des revendications 16 à 23, **caractérisé par le fait que** les premier et deuxième polymères filmogènes sont choisis, indifféremment l'un de l'autre, dans le groupe formé par les polymères vinyliques, les polyuréthanes, les polyesters, les polymères cellulosiques.

25. Procédé selon l'une quelconque des revendications 16 à 24, **caractérisé par le fait que** la teneur en respectivement premier et deuxième polymère filmogène va de 1 % à 70 % en poids, par rapport au poids total respectivement de la première et deuxième composition, et de préférence de 10 % à 40 % en poids.

26. Procédé selon l'une quelconque des revendications 16 à 25, **caractérisé par le fait que** la première et/ou la deuxième composition comprennent en outre au moins une matière colorante choisie dans le groupe formé par les colorants et les matières colorantes pulvérulentes ayant une taille de particule inférieure ou égale à 70 µm.

27. Procédé selon l'une quelconque des revendications 16 à 26, **caractérisé par le fait que** la première composition comprend une matière colorante consistant en un colorant et/ou les matières colorantes pulvérulentes ayant une taille de particule inférieure ou égale à 70 µm.

28. Procédé selon l'une quelconque des revendications 16 à 27, **caractérisé par le fait que** la deuxième composition comprend un milieu sensiblement translucide.

29. Procédé selon l'une quelconque des revendications 16 à 28, **caractérisé par le fait que** la première et/ou la deuxième composition sont appliquées sur un support choisi parmi les ongles, les joues, les paupières, le corps.

30. Kit de maquillage comprenant
- une première composition comprenant au moins un premier polymère filmogène dans un milieu cosmétiquement acceptable, et
- une deuxième composition comprenant au moins un deuxième polymère filmogène dans un milieu cosmétiquement acceptable, et au moins une matière colorante comprenant au moins un fragment de film de polymère coloré présentant deux faces sensiblement planes, ayant une épaisseur allant de 40 à 200 µm et dont la plus grande dimension va de 0,1 à 4 mm, le dit fragment étant insoluble dans le milieu de la composition,
la première composition ne comprenant pas de fragment de polymère comme présent dans la deuxième composition.

31. Kit de maquillage selon la revendication 30, **caractérisé par le fait que** le fragment de film a une épaisseur allant de 70 µm à 130 µm, et mieux de 90 µm à 110 µm.

32. Kit de maquillage selon l'une des revendications 30 ou 31, **caractérisé par le fait que** le fragment de film a une brillance, mesurée à l'aide d'un brillancemètre BYK-GARDNER à un angle de faisceau lumineux de 60°, inférieure ou égale à 15, et de préférence allant de 3 à 10.

33. Kit de maquillage selon l'une quelconque des revendications 30 à 32, **caractérisé par le fait que** la plus grande dimension du fragment de film coloré va de 0,1 mm à 2 mm, et mieux de 0,1 mm à 1 mm.

34. Kit de maquillage selon l'une quelconque des revendications 30 à 33, **caractérisé par le fait que** le fragment de film coloré est issu d'un film de polymère choisi dans le groupe formé par les polymères acryliques, les polymères d'acétate de polyvinyle, les copolymères styrène-acrylique, vinyle/versatate, vinyle/éthylène, les terpolymères vinyle/versatate/acrylate ou vinyle/éthylène/chlorure.

35. Kit de maquillage selon l'une quelconque des revendications 30 à 34, **caractérisé par le fait que** le fragment de film est présent en une teneur allant de 0,5 % à 30 % en poids, par rapport au poids total de la deuxième composition, et de préférence de 3 % à 20 % en poids.

36. Kit de maquillage selon l'une quelconque des revendications 30 à 35, **caractérisé par le fait que** la première et/ou la deuxième composition comprennent un milieu solvant organique ou un milieu aqueux.

37. Kit de maquillage selon l'une quelconque des revendications 30 à 36, **caractérisé par le fait que** les premier et deuxième polymères filmogènes sont choisis, indifféremment l'un de l'autre, dans le groupe formé par les polymères radicalaires, les polycondensats et les polymères d'origine naturelle.

38. Kit de maquillage selon l'une quelconque des revendications 30 à 37, **caractérisé par le fait que** les premier et deuxième polymères filmogènes sont choisis, indifféremment l'un de l'autre, dans le groupe formé par les polymères vinyliques, les polyuréthanes, les polyesters, les polymères cellulosiques.

39. Kit de maquillage selon l'une quelconque des revendications 30 à 38, **caractérisé par le fait que** la teneur en respectivement premier et deuxième polymère filmogène va de 1 % à 70 % en poids, par rapport au poids total respectivement de la première et deuxième composition, et de préférence de 10 % à 40 % en poids.

40. Kit de maquillage selon l'une quelconque des revendications 30 à 39, **caractérisé par le fait que** la première et/ou la deuxième composition comprennent en outre au moins une matière colorante choisie dans le groupe formé par les colorants et les matières colorantes pulvérulentes ayant une taille de particule inférieure ou égale à 70 µm.

41. Kit de maquillage selon l'une quelconque des revendications 30 à 40, **caractérisé par le fait que** la première composition comprend une matière colorante consistant en un colorant et/ou les matières colorantes pulvérulentes ayant une taille de particule inférieure ou égale à 70 µm.

42. Kit de maquillage selon l'une quelconque des revendications 30 à 41, **caractérisé par le fait que** la deuxième composition comprend un milieu sensiblement translucide.

43. Kit de maquillage selon l'une quelconque des revendications 30 à 42, **caractérisé par le fait que** la première et/ou deuxième composition comprennent en outre au moins un additif choisi dans le groupe formé par les agents auxiliaires de filmification, les agents épaississants, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

44. Kit de maquillage selon l'une quelconque des revendications 30 à 43, **caractérisé par le fait que** la première et/ou deuxième composition sont sous la forme de vernis à ongles, de fard à joue ou à paupières, de fond de teint, de produit de maquillage des lèvres ou du corps.

45. Kit de maquillage selon l'une quelconque des revendications 30 à 44, **caractérisé par le fait qu'**il contient des moyens pour appliquer sur les matières kératiniques la première et la deuxième compositions.

46. Support maquillé comprenant un maquillage susceptible d'être obtenu selon le procédé tel que défini selon l'une des revendications 15 à 29 appliqué sur ledit support.

47. Support selon la revendication 46, **caractérisé par le fait qu'**il se présente sous forme de faux ongles, ou de postiches.

## Patentansprüche

1. Kosmetische Zusammensetzung zum Schminken, die in einem kosmetisch akzeptablen Medium ein filmbildendes Polymer und ein Farbmittel enthält, **dadurch gekennzeichnet, daß** das Farbmittel mindestens ein Filmfragment eines farbigen Polymers umfaßt, das zwei im wesentlichen plane Flächen hat, das eine Dicke im Bereich von 40 bis 200 µm aufweist und dessen größte Abmessung im Bereich von 0,1 bis 4 mm liegt, wobei das Fragment in dem Medium der Zusammensetzung unlöslich ist.

2. Zusammensetzung nach Anspruch 1, **dadurch gekennzeichnet, daß** das Filmfragment eine Dicke im Bereich von 70 bis 130 µm und noch besser 90 bis 110 µm aufweist.

3. Zusammensetzung nach Anspruch 1 oder 2, **dadurch gekennzeichnet, daß** das Filmfragment einen Glanz von höchstens 15 und vorzugsweise im Bereich von 3 bis 10 aufweist, wobei der Glanz unter einem Einfallswinkel von 60° mit einem Glanzmeßgerät BYK-GARDNER ermittelt wird.

4. Zusammensetzung nach einem der vorhergehenden Ansprüche, **dadurch gekennzeichnet, daß** die größte Abmessung des farbigen Filmfragments im Bereich von 0,1 bis 2 mm und besser 0,1 bis 1 mm liegt.

5. Zusammensetzung nach einem der vorhergehenden Ansprüche, **dadurch gekennzeichnet, daß** das farbige Filmfragment von dem Film eines Polymers abgeleitet ist, das unter den Acrylpolymeren, Polyvinylacetatpolymeren, Styrol/Acryl-Copolymeren, Vinyl/Versatat-Copolymeren, Vinyl/Ethylen-Copolymeren, Vinyl/Versatat/Acrylat-Terpolymeren oder Vinyl/Ethylen/Chlorid-Terpolymeren ausgewählt ist.

6. Zusammensetzung nach einem der vorhergehenden Ansprüche, **dadurch gekennzeichnet, daß** das Filmfragment in einer Menge von 0,5 bis 30 Gew.-%, bezogen auf das Gesamtgewicht der Zusammensetzung, und vorzugsweise 3 bis 20 Gew.-% vorliegt.

7. Zusammensetzung nach einem der vorhergehenden Ansprüche, **dadurch gekennzeichnet, daß** sie ein organisches Lösungsmittelmedium oder ein wäßriges Medium enthält.

8. Zusammensetzung nach einem der vorhergehenden Ansprüche, **dadurch gekennzeichnet, daß** das filmbildende Polymer unter den durch radikalische Polymerisation hergestellten Polymeren, Polykondensaten und Polymeren natürlicher Herkunft ausgewählt ist.

9. Zusammensetzung nach einem der vorhergehenden Ansprüche, **dadurch gekennzeichnet, daß** das filmbildende Polymer unter den Vinylpolymeren, Polyurethanen, Polyestern und Cellulosepolymeren ausgewählt ist.

10. Zusammensetzung nach einem der vorhergehenden Ansprüche, **dadurch gekennzeichnet, daß** der Mengenanteil des filmbildenden Polymers im Bereich von 1 bis 70 Gew.-%, bezogen auf das Gesamtgewicht der Zusammensetzung, und vorzugsweise im Bereich von 10 bis 40 Gew.-% liegt.

11. Zusammensetzung nach einem der vorhergehenden Ansprüche, **dadurch gekennzeichnet, daß** sie ferner ein zusätzliches Farbmittel enthält, das von dem farbigen Filmfragment verschieden ist.

12. Zusammensetzung nach Anspruch 11, **dadurch gekennzeichnet, daß** das zusätzliche Farbmittel unter den Farbstoffen und pulverförmigen Farbmitteln mit einer Partikelgröße von höchstens 70 µm ausgewählt ist.

13. Zusammensetzung nach einem der vorhergehenden Ansprüche, **dadurch gekennzeichnet, daß** sie ferner mindestens einen Zusatzstoff enthält, der ausgewählt ist unter: Hilfsstoffen für die Filmbildung, Verdickungsmitteln, Spreitmitteln, Netzmitteln, Dispergiermitteln, Antischaummitteln, Konservierungsmitteln, UV-Filtern, Wirkstoffen, grenzflächenaktiven Stoffen, Hydratisierungsmitteln, Parfums, Neutralisationsmitteln, Stabilisierungsmitteln und Antioxidantien.

14. Zusammensetzung nach einem der vorhergehenden Ansprüche, **dadurch gekennzeichnet, daß** sie als Nagellack, Wangenrouge, Lidschatten, Make-up oder Produkt zum Schminken des Körpers vorliegt.

15. Kosmetisches Verfahren zum Schminken von Keratinsubstanzen, das darin besteht, auf die Keratinsubstanzen mindestens eine Schicht einer Zusammensetzung nach einem der vorhergehenden Ansprüche aufzutragen.

16. Kosmetisches Verfahren zum Schminken von Keratinsubstanzen, **dadurch gekennzeichnet, daß** auf die Keratinsubstanzen aufgetragen wird:
- eine erste Schicht einer ersten Zusammensetzung, die in einem kosmetisch akzeptablen Medium ein erstes filmbildendes Polymer enthält, und
- dann auf zumindest einem Teil der ersten Schicht eine zweite Schicht einer zweiten kosmetischen Zusammensetzung, die in einem kosmetisch akzeptablen Medium mindestens ein zweites filmbildendes Polymer und mindestens ein Farbmittel enthält, das mindestens ein Filmfragment eines farbigen Polymers umfaßt, das zwei im wesentlichen plane Flächen hat, das eine Dicke im Bereich von 40 bis 200 µm aufweist und dessen größte Abmessung im Bereich von 0,1 bis 4 mm liegt, wobei das Fragment in dem Medium der Zusammensetzung unlöslich ist,
- wobei die erste Zusammensetzung kein Polymerfragment, wie es in der zweiten Zusammensetzung vorliegt, enthält.

17. Verfahren nach Anspruch 16, **dadurch gekennzeichnet, daß** das Filmfragment eine Dicke im Bereich von 70 bis 130 µm und noch besser 90 bis 110 µm aufweist.

18. Verfahren nach Anspruch 16 oder 17, **dadurch gekennzeichnet, daß** das Filmfragment einen Glanz von höchstens 15 und vorzugsweise im Bereich von 3 bis 10 aufweist, wobei der Glanz unter einem Einfallswinkel von 60° mit einem Glanzmeßgerät BYK-GARDNER ermittelt wird.

19. Verfahren nach einem der Ansprüche 16 bis 18, **dadurch gekennzeichnet, daß** die größte Abmessung des farbigen Filmfragments im Bereich von 0,1 bis 2 mm und besser 0,1 bis 1mm liegt.

20. Verfahren nach einem der Ansprüche 16 bis 19, **dadurch gekennzeichnet, daß** das farbige Filmfragment von dem Film eines Polymers abgeleitet ist, das unter den Acrylpolymeren, Polyvinylacetatpolymeren, Styrol/Acryl-Copolymeren, Vinyl/Versatat-Copolymeren, Vinyl/Ethylen-Copolymeren, Vinyl/Versatat/Acrylat-Terpolymeren oder Vinyl/Ethylen/Chlorid-Terpolymeren ausgewählt ist.

21. Verfahren nach einem der Ansprüche 16 bis 20, **dadurch gekennzeichnet, daß** das Filmfragment in einer Menge von 0,5 bis 30 Gew.-%, bezogen auf das Gesamtgewicht der Zusammensetzung, und vorzugsweise 3 bis 20 Gew.-% vorliegt.

22. Verfahren nach einem der Ansprüche 16 bis 21, **dadurch gekennzeichnet, daß** die erste und/oder zweite Zusammensetzung ein organisches Lösungsmittelmedium oder ein wäßriges Medium enthält.

23. Verfahren nach einem der Ansprüche 16 bis 22, **dadurch gekennzeichnet, daß** das erste und zweite filmbildende Polymer unabhängig voneinander unter den durch radikalische Polymerisation hergestellten Polymeren, Polykondensaten und Polymeren natürlicher Herkunft ausgewählt sind.

24. Verfahren nach einem der Ansprüche 16 bis 23, **dadurch gekennzeichnet, daß** das erste und zweite filmbildende Polymer unabhängig voneinander unter den Vinylpolymeren, Polyurethanen, Polyestern und Cellulosepolymeren ausgewählt sind.

25. Verfahren nach einem der Ansprüche 16 bis 24, **dadurch gekennzeichnet, daß** der Mengenanteil des ersten bzw. zweiten filmbildenden Polymers im Bereich von 1 bis 70 Gew.-%, bezogen auf das Gesamtgewicht der Zusammensetzung, und vorzugsweise im Bereich von 10 bis 40 Gew.-% liegt.

26. Verfahren nach einem der Ansprüche 16 bis 25, **dadurch gekennzeichnet, daß** die erste und/oder zweite Zusammensetzung ferner ein zusätzliches Farbmittel enthält, das unter den Farbstoffen und pulverförmigen Farbmitteln mit einer Partikelgröße von höchstens 70 µm ausgewählt ist.

27. Verfahren nach einem der Ansprüche 16 bis 26, **dadurch gekennzeichnet, daß** die erste Zusammensetzung ein Farbmittel enthält, das aus Farbstoffen und/oder pulverförmigen. Farbmitteln mit einer Partikelgröße von höchstens 70 µm besteht.

28. Verfahren nach einem der Ansprüche 16 bis 27, **dadurch gekennzeichnet, daß** die zweite Zusammensetzung ein im wesentlichen durchscheinendes Medium enthält.

29. Verfahren nach einem der Ansprüche 16 bis 28, **dadurch gekennzeichnet, daß** die erste und/oder zweite Zusammensetzung auf einen Träger aufgetragen werden, der unter den Nägeln, Wangen, Lidern und dem Körper ausgewählt ist.

30. Kit zum Schminken, der enthält:
- eine erste Zusammensetzung, die in einem kosmetisch akzeptablen Medium mindestens ein erstes filmbildendes Polymer enthält, und
- eine zweite Zusammensetzung, die in einem kosmetisch akzeptablen Medium mindestens ein zweites filmbildendes Polymer und ein Farbmittel enthält, das mindestens ein Filmfragment eines farbigen Polymers umfaßt, das zwei im wesentlichen plane Flächen hat, das eine Dicke im Bereich von 40 bis 200 µm aufweist und dessen größte Abmessung im Bereich von 0,1 bis 4 mm liegt, wobei das Fragment in dem Medium der Zusammensetzung unlöslich ist,
- wobei die erste Zusammensetzung kein Polymerfragment, wie es in der zweiten Zusammensetzung vorliegt, enthält.

31. Kit zum Schminken nach Anspruch 30, **dadurch gekennzeichnet, daß** das Filmfragment eine Dicke im Bereich von 70 bis 130 µm und noch besser 90 bis 110 µm aufweist.

32. Kit zum Schminken nach einem der Ansprüche 30 und 31, **dadurch gekennzeichnet, daß** das Filmfragment einen Glanz von höchstens 15 und vorzugsweise im Bereich von 3 bis 10 aufweist, wobei der Glanz unter einem Einfallswinkel von 60° mit einem Glanzmeßgerät BYK-GARDNER ermittelt wird.

33. Kit zum Schminken nach einem der Ansprüche 30 bis 32, **dadurch gekennzeichnet, daß** die größte Abmessung des farbigen Filmfragments im Bereich von 0,1 bis 2 mm und besser 0,1 bis 1mm liegt.

34. Kit zum Schminken nach einem der Ansprüche 30 bis 33, **dadurch gekennzeichnet, daß** das farbige Filmfragment von dem Film eines Polymers abgeleitet ist, das unter den Acrylpolymeren, Polyvinylacetatpolymeren, Styrol/Acryl-Copolymeren, Vinyl/Versatat-Copolymeren, Vinyl/Ethylen-Copolymeren, Vinyl/Versatat/Acrylat-Terpolymeren oder Vinyl/Ethylen/Chlorid-Terpolymeren ausgewählt ist.

35. Kit zum Schminken nach einem der Ansprüche 30 bis 34, **dadurch gekennzeichnet, daß** das Filmfragment in einer Menge von 0,5 bis 30 Gew.-%, bezogen auf das Gesamtgewicht der zweiten Zusammensetzung, und vorzugsweise 3 bis 20 Gew.-% vorliegt.

36. Kit zum Schminken nach einem der Ansprüche 30 bis 35, **dadurch gekennzeichnet, daß** die erste und/oder zweite Zusammensetzung ein organisches Lösungsmittelmedium oder ein wäßriges Medium enthalten.

37. Kit zum Schminken nach einem der Ansprüche 30 bis 36, **dadurch gekennzeichnet, daß** das erste und zweite filmbildende Polymer unabhängig voneinander unter den durch radikalische Polymerisation hergestellten Polymeren, Polykondensaten und Polymeren natürlicher Herkunft ausgewählt ist.

38. Kit zum Schminken nach einem der Ansprüche 30 bis 37, **dadurch gekennzeichnet, daß** das erste und zweite filmbildende Polymer unter den Vinylpolymeren, Polyurethanen, Polyestern und Cellulosepolymeren ausgewählt ist.

39. Kit zum Schminken nach einem der Ansprüche 30 bis 38, **dadurch gekennzeichnet, daß** der Mengenanteil des ersten bzw. zweiten filmbildenden Polymers im Bereich von 1 bis 70 Gew.-%, bezogen auf das Gesamtgewicht der ersten bzw. zweiten Zusammensetzung, und vorzugsweise im Bereich von 10 bis 40 Gew.-% liegt.

40. Kit zum Schminken nach einem der Ansprüche 30 bis 39, **dadurch gekennzeichnet, daß** die erste und/oder zweite Zusammensetzung ferner ein zusätzliches Farbmittel enthält, das unter den Farbstoffen und pulverförmigen Farbmitteln mit einer Partikelgröße von höchstens 70 µm ausgewählt ist.

41. Kit zum Schminken nach einem der Ansprüche 30 bis 40, **dadurch gekennzeichnet, daß** die erste Zusammensetzung ein Farbmittel enthält, das aus Farbstoffen und/oder pulverförmigen Farbmitteln mit einer Partikelgröße von höchstens 70 µm besteht.

42. Kit zum Schminken nach einem der Ansprüche 30 bis 41, **dadurch gekennzeichnet, daß** die zweite Zusammensetzung ein im wesentlichen durchscheinendes Medium enthält.

43. Kit zum Schminken nach einem der Ansprüche 30 bis 42, **dadurch gekennzeichnet, daß** die erste und/oder zweite Zusammensetzung außerdem mindestens einen Zusatzstoff enthält, der ausgewählt ist unter: Hilfsstoffen für die Filmbildung, Verdickungsmitteln, Spreitmitteln, Netzmitteln, Dispergiermitteln, Antischaummitteln, Konservierungsmitteln, UV-Filtern, Wirkstoffen, grenzflächenaktiven Stoffen, Hydratisierungsmitteln, Parfums, Neutralisationsmitteln, Stabilisierungsmitteln und Antioxidantien.

44. Kit zum Schminken nach einem der Ansprüche 30 bis 43, **dadurch gekennzeichnet, daß** die erste und/oder zweite Zusammensetzung als Nagellack, Wangenrouge, Lidschatten, Make-up oder Produkt zum Schminken der Lippen oder des Körpers vorliegt.

45. Kit zum Schminken nach einem der Ansprüche 30 bis 44, **dadurch gekennzeichnet, daß** er Mittel enthält, um die erste und zweite Zusammensetzung auf die Keratinsubstanzen aufzutragen.

46. Geschminkter Träger, der eine Schminke aufweist, die nach dem Verfahren nach einem der Ansprüche 15 bis 29 erzeugt werden kann und auf den Träger aufgetragen wird.

47. Träger nach Anspruch 46, **dadurch gekennzeichnet, daß** er in Form von künstlichen Nägeln oder Schönheitspflastern vorliegt.

## Claims

1. Cosmetic make-up composition comprising, in a cosmetically acceptable medium, a film-forming polymer and a colouring material, **characterized in that** the colouring material comprises at least one coloured polymer film fragment which exhibits two substantially flat faces, which has a thickness ranging from 40 to 200 µm and which has a larger dimension ranging from 0.1 to 4 mm, the said fragment being insoluble in the medium of the composition.

2. Composition according to Claim 1, **characterized in that** the film fragment has a thickness ranging from 70 µm to 130 µm and better still from 90 µm to 110 µm.

3. Composition according to Claim 1 or 2, **characterized in that** the film fragment has a gloss, measured using a Byk-Gardner glossmeter at a light beam angle of 60°, of less than or equal to 15 and preferably ranging from 3 to 10.

4. Composition according to any one of the preceding claims, **characterized in that** the larger dimension of the coloured film fragment ranges from 0.1 mm to 2 mm and better still from 0.1 mm to 1 mm.

5. Composition according to any one of the preceding claims, **characterized in that** the coloured film fragment results from a polymer film chosen from the group formed by acrylic polymers, poly(vinyl acetate) polymers, styrene-acrylic, vinyl/versatate and vinyl/ethylene copolymers, and vinyl/versatate/acrylate and vinyl/ethylene/chloride terpolymers.

6. Composition according to any one of the preceding claims, **characterized in that** the film fragment is present at a content ranging from 0,5% to 30% by weight with respect to the total weight of the composition and preferably from 3% to 20% by weight.

7. Composition according to any one of the preceding claims, **characterized in that** it comprises an organic solvent medium or an aqueous medium.

8. Composition according to any one of the preceding claims, **characterized in that** the film-forming polymer is chosen from the group formed by radical polymers, polycondensates and polymers of natural origin.

9. Composition according to any one of the preceding claims, **characterized in that** the film-forming polymer is chosen from the group formed by vinyl polymers, polyurethanes, polyesters and cellulose polymers.

10. Composition according to any one of the preceding claims, **characterized in that** the content of film-forming polymer ranges from 1% to 70% by weight with respect to the total weight of the composition and preferably from 10% to 40% by weight.

11. Composition according to any one of the preceding claims, **characterized in that** it furthermore comprises an additional colouring material other than that of the coloured film fragment.

12. Composition according to Claim 11, **characterized in that** the additional colouring material is chosen from dyes and pulverulent colouring materials having a particle size of less than or equal to 70 µm.

13. Composition according to any one of the preceding claims, **characterized in that** it additionally comprises at least one additive chosen from the group formed by additional agents which are able to form a film, thickening agents, spreading agents, wetting agents, dispersing agents, anti-foaming agents, preservatives, UV screening agents, active principles, surfactants, moisturizing agents, fragrances, neutralizing agents, stabilizing agents and antioxidants.

14. Composition according to any one of the preceding claims, **characterized in that** it is provided in the form of a nail varnish, face powder, eye shadow, foundation or product for making up the body.

15. Cosmetic process for making up keratinous substances which consists in applying, to the keratinous substances, at least one layer of a composition according to any one of the preceding claims.

16. Cosmetic process for making up keratinous substances, **characterized in that** the following are applied to the keratinous substances:
- a first layer of a first composition comprising, in a cosmetically acceptable medium, a first film-forming polymer,
- and then, to at least a portion of the said first layer, a second layer of a second composition comprising at least one second film-forming polymer in a cosmetically acceptable medium and at least one colouring material comprising at least one coloured polymer film fragment which exhibits two substantially flat faces, which has a thickness ranging from 40 to 200 µm and which has a larger dimension ranging from 0.1 to 4 mm, the said fragment being insoluble in the medium of the composition,
the first composition not comprising a polymer fragment as present in the second composition.

17. Process according to Claim 16, **characterized in that** the film fragment has a thickness ranging from 70 µm to 130 µm and better still from 90 µm to 110 µm.

18. Process according to Claim 16 or 17, **characterized in that** the film fragment has a gloss, measured using a Byk-Gardner glossmeter at a light beam angle of 60, ° of less than or equal to 15 and preferably ranging from 3 to 10.

19. Process according to any one of Claims 16 to 18, **characterized in that** the larger dimension of the coloured film fragment ranges from 0.1 mm to 2 mm and better still from 0.1 mm to 1 mm.

20. Process according to any one of Claims 16 to 19, **characterized in that** the coloured film fragment results from a polymer film chosen from the group formed by acrylic polymers, poly(vinyl acetate) polymers, styrene-acrylic, vinyl/versatate and vinyl/ethylene copolymers, and vinyl/versatate/acrylate and vinyl/ethylene/chloride terpolymers.

21. Process according to any one of Claims 16 to 20, **characterized in that** the film fragment is present at a content ranging from 0.5% to 30% by weight with respect to the total weight of the second composition and preferably from 3% to 20% by weight.

22. Process according to any one of Claims 16 to 21, **characterized in that** the first and/or the second composition comprise an organic solvent medium or an aqueous medium.

23. Process according to any one of Claims 16 to 22, **characterized in that** the first and second film-forming polymers are chosen, without distinction from one another, from the group formed by radical polymers, polycondensates and polymers of natural origin.

24. Process according to any one of Claims 16 to 23, **characterized in that** the first and second film-forming polymexs are chosen, without distinction from one another, from the group formed by vinyl polymers, polyurethanes, polyesters and cellulose polymers.

25. Process according to any one of Claims 16 to 24, **characterized in that** the content of respectively first and second film-forming polymer ranges from 1% to 70% by weight with respect to the total weight respectively of the first and second composition and preferably from 10% to 40% by weight.

26. Process according to any one of Claims 16 to 25, **characterized in that** the first and/or the second composition additionally comprise at least one colouring material chosen from the group formed by dyes and pulverulent colouring materials having a particle size of less than or equal to 70 µm.

27. Process according to any one of Claims 16 to 26, **characterized in that** the first composition comprises a colouring material consisting of a dye and/or pulverulent colouring materials having a particle size of less than or equal to 70 µm.

28. Process according to any one of Claims 16 to 27, **characterized in that** the second composition comprises a substantially translucent medium.

29. Process according to any one of Claims 16 to 28, **characterized in that** the first and/or the second composition are applied to a substrate chosen from the nails, cheeks, eyelids or body.

30. Make-up kit comprising:
- a first composition comprising at least one first film-forming polymer in a cosmetically acceptable medium, and
- a second composition comprising at least one second film-forming polymer in a cosmetically acceptable medium and at least one colouring material comprising at least one coloured polymer film fragment which exhibits two substantially flat faces, which has a thickness ranging from 40 to 200 µm and which has a larger dimension ranging from 0.1 to 4 mm, the said fragment being insoluble in the medium of the composition,
the first composition not comprising a polymer fragment as present in the second composition.

31. Make-up kit according to Claim 30, **characterized in that** the film fragment has a thickness ranging from 70 µm to 130 µm and better still from 90 µm to 110 µm.

32. Make-up kit according to either of Claims 30 and 31, **characterized in that** the film fragment has a gloss, measured using a Byk-Gardner glossmeter at a light beam angle of 60°, of less than or equal to 15 and preferably ranging from 3 to 10.

33. Make-up kit according to any one of Claims 30 to 32, **characterized in that** the larger dimension of the coloured film fragment ranges from 0.1 mm to 2 mm and better still from 0.1 mm to 1 mm.

34. Make-up kit according to any one of Claims 30 to 33, **characterized in that** the coloured film fragment results from a polymer film chosen from the group formed by acrylic polymers, poly(vinyl acetate) polymers, styrene-acrylic, vinyl/versatate and vinyl/ethylene copolymers, and vinyl/versatate/acrylate and vinyl/ethylene/chloride terpolymers.

35. Make-up kit according to any one of Claims 30 to 34, **characterized in that** the film fragment is present at a content ranging from 0.5% to 30% by weight with respect to the total weight of the second composition and preferably from 3% to 20% by weight.

36. Make-up kit according to any one of Claims 30 to 35, **characterized in that** the first and/or the second composition comprise an organic solvent medium or an aqueous medium.

37. Make-up kit according to any one of Claims 30 to 36, **characterized in that** the first and second film-forming polymers are chosen, without distinction from one another, from the group formed by radical polymers, polycondensates and polymers of natural origin.

38. Make-up kit according to any one of Claims 30 to 37, **characterized in that** the first and second film-forming polymers are chosen, without distinction from one another, from the group formed by vinyl polymers, polyurethanes, polyesters and cellulose polymers.

39. Make-up kit according to any one of Claims 30 to 38, **characterized in that** the content of respectively first and second film-forming polymer ranges from 1% to 70% by weight with respect to the total weight respectively of the first and second composition and preferably from 10% to 40% by weight.

40. Make-up kit according to any one of Claims 30 to 39, **characterized in that** the first and/or the second composition additionally comprise at least one colouring material chosen from the group formed by dyes and pulverulent colouring materials having a particle size of less than or equal to 70 µm.

41. Make-up kit according to any one of Claims 30 to 40, **characterized in that** the first composition comprises a colouring material consisting of a dye and/or pulverulent colouring materials having a particle size of less than or equal to 70 µm.

42. Make-up kit according to any one of Claims 30 to 41, **characterized in that** the second composition comprises a substantially translucent medium.

43. Make-up kit according to any one of Claims 30 to 42, **characterized in that** the first and/or the second composition additionally comprise at least one additive chosen from the group formed by additional agents which are able to form a film, thickening agents, spreading agents, wetting agents, dispersing agents, anti-foaming agents, preservatives, UV screening agents, active principles, surfactants, moisturizing agents, fragrances, neutralizing agents, stabilizing agents and antioxidants.

44. Make-up kit according to any one of Claims 30 to 43, **characterized in that** the first and/or the second composition are in the form of a nail varnish, face powder, eye shadow, foundation or product for making up the lips or body.

45. Make-up kit according to any one of Claims 30 to 44, **characterized in that** it comprises means for applying the first and the second compositions to keratinous substances.

46. Made-up substrate comprising a make-up capable of being obtained according to the process as defined according to one of Claims 15 to 29 applied to the said substrate.

47. Substrate according to Claim 46, **characterized in that** it is provided in the form of false nails or of postiches.
